# EUROPÄISCHE PATENTANMELDUNG

(11) **EP 3 549 518 A1**
(43) Veröffentlichungstag der Anmeldung: **09.10.2019**
(21) Anmeldenummer: 18165620.8
(22) Anmeldetag: 04.04.2018
(51) Int. Cl.: A61B 5/0215, A61F 2/848

(54) **HALTESTRUKTUR FÜR EIN IMPLANTAT**

(71) Anmelder: BIOTRONIK SE & Co. KG, 12359 Berlin (DE)
(72) Erfinder: Ulmer, Jens, 8008 Zürich (CH); Degen, Nicolas, 8222 Beringen (CH)
(74) Vertreter: Keck, Hans-Georg

(57) **Zusammenfassung**

Die Offenbarung betrifft ein Implantat (3), aufweisend einen Implantatkörper (3a) sowie eine Haltestruktur (10) zum Verankern des Implantats in einem Gefäß (6) eines Patienten, wobei die Haltestruktur (10) dazu konfiguriert ist aus einem komprimierten Zustand in einen expandierten Zustand gebracht zu werden. Es ist vorgesehen, dass die Haltestruktur (10) ein Hauptgerüst (1) sowie mit dem Hauptgerüst (1) verbundene Seitenarme (2) aufweist.

## Beschreibung

Die Offenbarung betrifft ein Implantat mit einer Haltestruktur zum Fixieren des Implantats, insbesondere eines Drucksensors, innerhalb der Lungenarterie.

Stentartige Systeme erlauben immer nur eine geringe Varianz des Zielgefäßdurchmessers (z. B. Lungenarterie), um sicher platziert werden zu können. Um eine breite Patientenpopulation zu versorgen, bedarf es somit immer verschiedener Größen des Implantats bzw. der Haltestruktur. Weiterhin ist eine Versorgung von stark gewundenen Gefäßen mit Stents nicht immer möglich, da diese immer versteifend und begradigend auf das Gefäß wirken. Weiterhin kann es mit Stents bei Gefäßverzweigungen zu einem Verschluss eines Gefäßastes durch störende Stentstreben kommen. Dies kann nur durch ein Aufweiten der Stentstreben mittels Ballondilatation, d. h., mit Inkaufnahme einer Strukturschwächung des Stents abgemildert werden.

Drahtschlaufen hingegen bieten hier Abhilfe, sind aber für größere (aktive) Implantate aufgrund der geringen Haltekraft weniger gut geeignet.

Das Dokument EP 2 997 933 B1 beschreibt eine implantierbare vaskuläre Vorrichtung mit einer Vielzahl an Verankerungselementen mit Durchstechelementen. Weiterhin beschreibt das Dokument US 7,572,228 B2 ein Implantat mit stentartigen oder wendelförmigen Haltestrukturen. Schließlich beschreibt das Dokument US 9,827,426 B2 ebenfalls ein Implantat mit einer wendelförmigen Haltestruktur.

Hiervon ausgehend liegt der vorliegenden Erfindung die Aufgabe zugrunde, ein Implantat mit einer Haltestruktur zu schaffen, die zur Fixierung des Implantats in Gefäßen mit einer großen Varianz der Gefäßdurchmesser eine hinreichend hohe Haltekraft bei gleichzeitiger hoher Flexibilität der Haltestruktur bereitstellt.

Diese Aufgabe wird durch ein Implantat mit den Merkmalen des Anspruchs 1 gelöst. Weitere Ausgestaltungen sind in den Unteransprüchen angegeben und werden nachfolgen detailliert beschrieben.

Gemäß Anspruch 1 wird ein Implantat offenbart, aufweisend einen Implantatkörper sowie eine Haltestruktur zum Verankern des Implantats in einem Gefäß eines Patienten, wobei die Haltestruktur dazu konfiguriert ist, aus einem komprimierten Zustand in einen expandierten Zustand gebracht zu werden. Es ist vorgesehen, dass die Haltestruktur ein Hauptgerüst sowie mit dem Hauptgerüst verbundene Seitenarme aufweist.

Gemäß einer Ausführungsform ist vorgesehen, dass die Haltestruktur eine offene Haltestruktur ist, wobei insbesondere die Seitenarme jeweils nicht geschlossen umlaufen, d. h., jeweils nicht ringförmig, sondern offen ausgebildet sind.

Weiterhin ist gemäß einer Ausführungsform vorgesehen, dass der jeweilige Seitenarm in dem expandierten Zustand der Haltestruktur einen Kreisbogen bildet, oder dass in dem expandierten Zustand eine Projektion des jeweiligen Seitenarmes auf eine Ebene (insbesondere auf eine senkrecht zum Hauptgerüst orientierte Ebene oder auf eine Querschnittsebene des Gefäßes in einem implantierten Zustand des Implantats) einen Kreisbogen bildet.

Weiterhin ist gemäß einer Ausführungsform vorgesehen, dass die Haltestruktur eine Mehrzahl an Paaren von Seitenarmen aufweist, wobei in dem expandierten Zustand der Haltestruktur eine Projektion des jeweiligen Seitenarmpaares auf eine Ebene (insbesondere auf eine senkrecht zum Hauptgerüst orientierte Ebene oder auf eine Querschnittsebene des Gefäßes in einem implantierten Zustand des Implantats) einen Kreisbogen mit einem Mittelpunktswinkel von zumindest 180° bildet.

Weiterhin ist gemäß einer Ausführungsform vorgesehen, dass die beiden Seitenarme des jeweiligen Seitenarmpaares benachbarte Seitenarme sind, die versetzt zueinander vom Hauptgerüst abgehen. Aufgrund der versetzten Anordnung können die Seitenarme bei geringen Gefäßdurchmessern einander durchdringen bzw. sich aneinander vorbei erstrecken.

Weiterhin ist gemäß einer Ausführungsform vorgesehen, dass das Hauptgerüst ein proximales Hauptgerüstteil und ein distales Hauptgerüstteil aufweist, wobei eine Anzahl der Seitenarme mit dem proximalen Hauptgerüstteil verbunden ist und von diesem absteht, und wobei eine restliche Anzahl der Seitenarme mit dem distalen Hauptgerüstteil verbunden ist und von diesem absteht.

Das Implantat kann eine Vorzugsrichtung haben, beispielsweise entlang des Blutflusses in dem Gefäß. Im Sinne der vorliegenden Offenbarung bedeutet "proximal" eine Erstreckung einer solchermaßen gekennzeichneten Komponente oder eines solchermaßen gekennzeichneten Ortes entgegen der Vorzugsrichtung (z. B. entgegen des Blutflusses). Eine als "distal" bezeichnete Komponente bzw. ein als "distal" bezeichneter Ort bezeichnet eine Erstreckung entlang der Vorzugsrichtung (z. B. entlang des Blutflusses).

Weiterhin ist gemäß einer Ausführungsform vorgesehen, dass die beiden Hauptgerüstteile jeweils in einer Richtung vom Implantatkörper abstehen oder über den Implantatkörper hinausragen, wobei die beiden Richtungen unterschiedlich sind, insbesondere einander entgegengesetzt.

Weiterhin ist gemäß einer Ausführungsform vorgesehen, dass die mit dem proximalen Hauptgerüstteil verbundenen Seitenarme unter einem Winkel vom proximalen Hauptgerüstteil abstehen, der in einem Bereich von 45° bis 90° liegt (mathematisch positiver Drehsinn bzw. Linksdrehung, d. h. gegen den Uhrzeigersinn), und/oder dass die mit dem distalen Hauptgerüstteil verbundenen Seitenarme unter einem Winkel vom distalen Hauptgerüstteil abstehen, der in einem Bereich von -90° bis -45° liegt (mathematisch negativer Drehsinn bzw. Rechtsdrehung, d. h. im Uhrzeigersinn).

Weiterhin ist gemäß einer Ausführungsform vorgesehen, dass der Implantatkörper längserstreckt ausgebildet ist und sich entlang einer Längsachse erstreckt, wobei die beiden besagten Richtungen (siehe oben) insbesondere parallel zu der Längsachse verlaufen bzw. auf dieser liegen.

Weiterhin ist gemäß einer Ausführungsform vorgesehen, dass der Implantatkörper ein proximales Ende und ein distales Ende aufweist, wobei sich die beiden Enden insbesondere in Richtung der Längsachse einander gegenüberliegen, wobei die beiden Enden weiterhin insbesondere über einen mittleren Abschnitt des Implantatkörpers miteinander verbunden sind.

Weiterhin ist gemäß einer Ausführungsform vorgesehen, dass das proximale Hauptgerüstteil sowie das distale Hauptgerüstteil mit dem Implantatkörper verbunden sind. Die beiden Hauptgerüstteile des Hauptgerüstes können dabei separat ausgebildet sein und können lediglich über den Implantatkörper miteinander verbunden sein. Die beiden Hauptgerüstteile können jedoch auch ein einteiliges zusammenhängendes Hauptgerüst bilden.

Weiterhin ist gemäß einer Ausführungsform vorgesehen, dass das proximale Hauptgerüstteil mit dem proximalen Ende des Implantatkörper über eine Verbindungstelle verbunden ist und/oder dass das distale Hauptgerüstteil mit dem distalen Ende des Implantatkörper über eine Verbindungstelle verbunden ist.

Weiterhin ist gemäß einer Ausführungsform vorgesehen, dass die besagten Verbindungstellen in einer Ebene mit einer größten Seitenfläche des Implantatkörpers liegen.

Weiterhin ist gemäß einer alternativen Ausführungsform vorgesehen, dass das distale und das proximale Hauptgerüstteil über eine gemeinsame Verbindungstelle mit dem mittleren Abschnitt des Implantatkörpers verbunden sind.

Weiterhin ist gemäß einer Ausführungsform vorgesehen, dass der proximale Hauptgerüstteil durch einen längs erstreckten Arm gebildet ist, und/oder dass der distale Hauptgerüstteil durch einen längs erstreckten Arm gebildet ist. Die beiden Arme erstrecken sich jeweils insbesondere entlang der Längsachse bzw. in die beiden besagten (z. B. einander entgegen gesetzten) Richtungen.

Weiterhin ist gemäß einer Ausführungsform vorgesehen, dass das Hauptgerüst und/oder die Hauptgerüstteile und/oder die Seitenarme aus einem Metall gefertigt ist bzw. sind oder ein Metall aufweist bzw. aufweisen. Bei einem selbstexpandierenden Implantat kann das Metall Nitinol sein. Bei einem ballonexpandierendem Implantat kann das Metall Edelstahl (stainless steel 316L), Co-Cr (L605) oder Pt-Ir sein.

Weiterhin ist gemäß einer Ausführungsform vorgesehen, dass das Hauptgerüst und/oder die beiden Hauptgerüstteile jeweils aus einem Metalldraht gebildet sind oder einen Metalldraht aufweisen. Weiterhin ist gemäß einer Ausführungsform vorgesehen, dass der jeweilige Seitenarm aus einem Metalldraht gebildet ist oder einen Metalldraht aufweist. Bei dem Metall kann es sich um die oben genannten Metalle handeln. Die gesamte Haltestruktur kann auch einem Stent ähnlich in der slottet tube Technik hergestellt werden. Dieses stentartige Haltesystem kann anschließend mit dem Implantat durch eine Schweißnaht verbunden werden.

Weiterhin kann gemäß einer Ausführungsform vorgesehen sein, dass das Hauptgerüst der Haltestruktur durch den Implantatkörper selbst gebildet ist. In diesem Fall gehen die einzelnen Seitenarme vom Implantatkörper ab.

Weiterhin ist gemäß einer Ausführungsform vorgesehen, dass der jeweilige Seitenarm ein dem Hauptgerüst gegenüberliegendes Ende aufweist, wobei zumindest die Enden zweier benachbarter Seitenarme über eine Strebe miteinander verbunden sind.

Weiterhin ist gemäß einer Ausführungsform vorgesehen, dass die Enden je zweier benachbarter Seitenarme des proximalen Hauptgerüstteiles, die zur gleichen Seite hin vom proximalen Hauptgerüstteil abgehen, über eine Strebe miteinander verbunden sind.

Weiterhin sind gemäß einer Ausführungsform die Enden je zweier benachbarter Seitenarme des distalen Hauptgerüstteiles, die zur gleichen Seite hin vom distalen Hauptgerüstteil abgehen, über eine Strebe miteinander verbunden.

Weiterhin ist gemäß einer Ausführungsform vorgesehen, dass der Implantatkörper einen Drucksensor aufweist bzw. als Drucksensor ausgebildet ist. Des Weiteren kann der Implantatkörper einen Beschleunigungssensor, einen Lagesensor, einen Temperatursensor oder einen Biosensor (z. B. für Glukose) aufweisen oder als einer der vorgenannten Sensoren ausgebildet sein.

Weiterhin ist gemäß einer Ausführungsform vorgesehen, dass die Haltestruktur selbstexpandierend ausgebildet oder dass die Haltestruktur mittels eines Ballons expandierbar ist.

Weitere Merkmale und Ausführungsformen der vorliegenden Erfindung sollen nachfolgend anhand der Figuren erläutert werden. Es zeigen:
- Fig. 1: eine Aufsicht einer Ausführungsform eines Implantats mit einer Haltestruktur;
- Fig. 2: eine Seitenansicht des in der Fig. 1 gezeigten Implantats;
- Fig. 3: eine Variante der Haltestruktur mit nur einer Verbindung des Hauptgerüstes mit dem Implantatkörper;
- Fig. 4: eine Variante des in der Fig. 1 gezeigten Implantats, bei der die Seitenarme mittels einer Metallstruktur bzw. Streben verbunden sind um insbesondere eine zu starke Gewebserosion nach der Implantation zu verhindern;
- Fig. 5: einen Längsschnitt einer Ausführungsform eines Implantates innerhalb eines Gefäßes;
- Fig. 6: eine Aufsicht (links) und eine Seitenansicht (rechts) einer Ausführungsform eines Implantats, bei dem die Seitenarme direkt mit dem Implantat verbunden sind; und
- Fig. 7: einen Querschnitt der Haltestruktur (ohne Implantatkörper) bei unterschiedlichen Gefäßdurchmessern (a. großer, b. kleiner Durchmesser). Die gegenseitig verschiebbaren Seitenarme erlauben es, unterschiedliche Durchmesser einzunehmen um das Implantat bzw. den Implantatkörper so sicher zu fixieren.

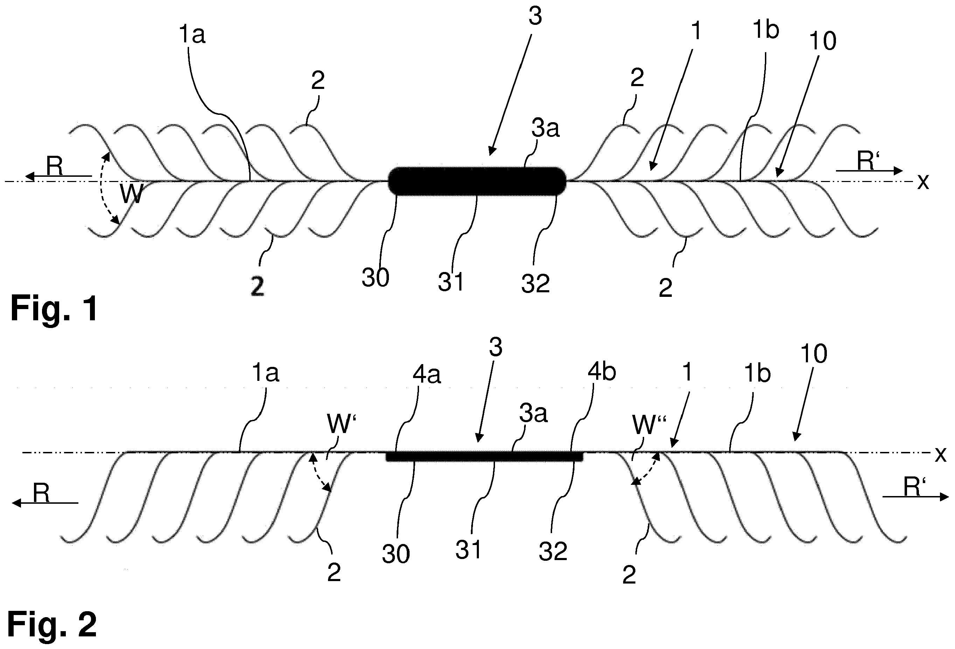

Für die Fixierung eines Implantat 3, hier beispielhaft eines aktiven Drucksensors in einer Pulmonalarterie 6, ist eine offene Haltestruktur 10 vorgesehen, die entlang eines Hauptgerüsts 1 eine Vielzahl an Seitenarmen 2 aufweist. Das Implantat 3 weist einen Implantatkörper 3a auf, der den eigentlichen Drucksensor aufweist und vorzugsweise mittig bezüglich des Hauptgerüsts 1 angeordnet ist. Gemäß der in den Figuren 1 und 2 dargestellten Ausführungsform des Implantats 3 weist das Hauptgerüst 1 entsprechend ein proximales Hauptgerüstteil 1a und ein distales Hauptgerüstteil 1b auf, die hier z. B. als längserstreckte Arme (z. B. aus einem Metalldraht) ausgebildet sind, die sich entlang einer Längsachse x des Implantatkörpers 3a erstrecken und insbesondere in entgegengesetzten Richtungen R, R' vom Implantatkörper 3 abstehen.

Ein aktiver Sensor ist ein Sensor, dessen Informationen durch eine aktive Funkstrecke übertragen werden. Daher benötigt der Sensor eine Energiequelle. Man kann sagen: Ein aktives Medizinprodukt (aktives Implantat) ist ein Medizinprodukt, dessen Betrieb von einer Stromquelle oder einer anderen Energiequelle (mit Ausnahme der direkt vom menschlichen Körper oder durch die Schwerkraft erzeugten Energie) abhängig ist. Hingegen beinhalten passive Sensoren keine eigene Energiequelle. Zum Auslesen der Informationen muss bei einem passiven Sensor Energie von außen in den Sensor übertragen werden (z. B. RFID).

Hierbei kann z. B. gemäß Figuren 1 und 2 vorgesehen sein, dass der proximale Hauptgerüstteil 1a über eine proximale Verbindungstelle 4a mit einem proximalen Ende 30 des Implantatkörpers verbunden ist, das über einen mittleren Abschnitt 31 des Implantatkörpers 3a mit einem distalen Ende 32 des Implantatkörpers 32 verbunden ist, wohingegen der distale Hauptgerüstteil 1b über eine distale Verbindungstelle 4b mit dem distalen Ende 32 des Implantatkörpers 3a verbunden ist. Vorzugsweise liegen die beiden Verbindungstellen 4a, 4b in einer Ebene mit der größten Seitenfläche des Implantatkörpers 3a.

Weiterhin sieht die in den Figuren 1 und 2 dargestellte Ausführungsform vor, dass die beiden Seitenarme 2 der Haltestruktur 10, die zu beiden Seiten hin von den Hauptgerüstteilen 1a, 1b abgehen, versetzt zueinander angeordnet sind. Aufgrund dieser versetzten Anordnung können die Seitenarme 2 bei geringen Gefäßdurchmessern einander durchdringen bzw. sich aneinander vorbei erstrecken.

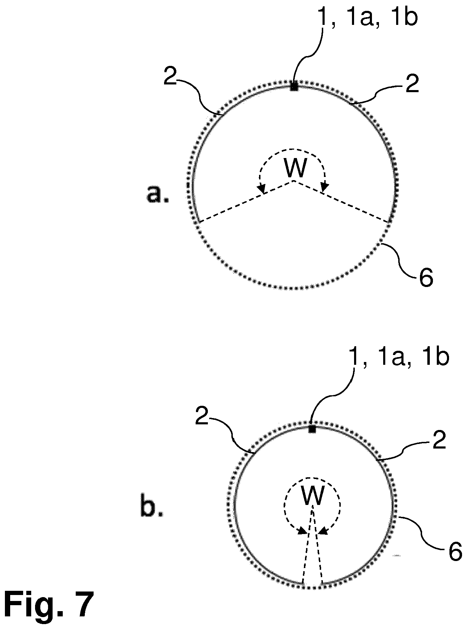

Benachbarte Seitenarme 2 des Hauptgerüsts 1 bzw. der Hauptgerüstteile 1a, 1b, die zu unterschiedlichen Seiten des Hauptgerüsts 1 hin vom Hauptgerüst 1 bzw. den Hauptgerüstteilen 1a, 1b abstehen, beschreiben in der Projektion gemäß Fig. 7 im freigesetzten Zustand des Implantats 3 bzw. der Haltestruktur 10 vorzugsweise einen Kreisbogen bzw. Halbkreis mit einem Mittelpunktswinkel W, der vorzugsweise zumindest 180° beträgt (vgl. Fig. 7a). Dieser Winkel gewährleistet eine sichere Fixierung des Implantats im Gefäß 6.

Weiterhin ist vorzugsweise vorgesehen, dass die Arme 2 dem proximalen Hauptgerüst 1a in einem Winkel W' zwischen 90° und 45° und dem distalen Hauptgerüst 1b in einem Winkel W" zwischen -90° und -45° entspringen.

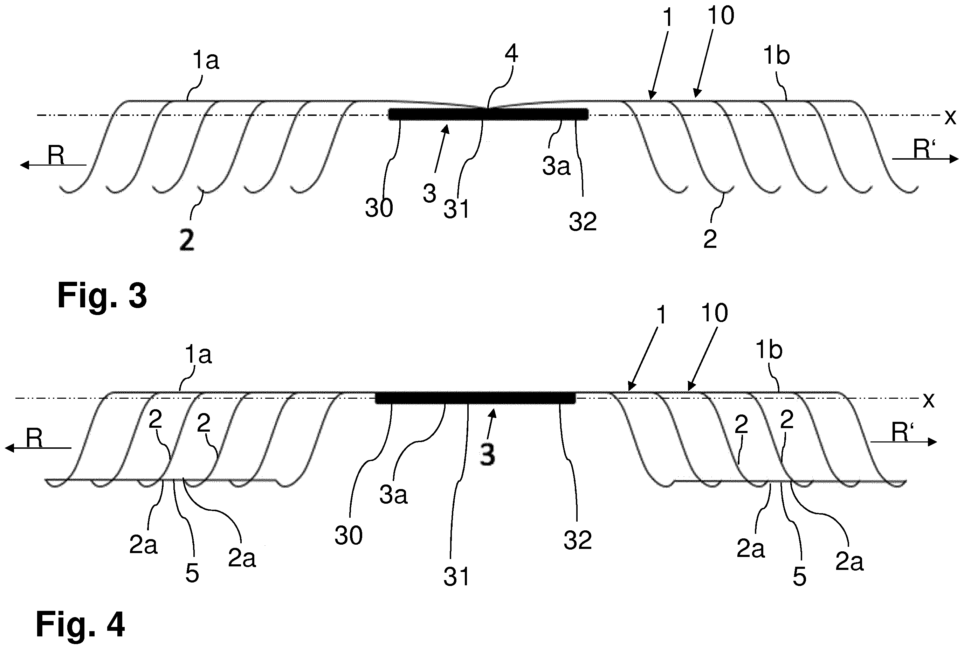

Figur 3 zeigt eine Ausführungsform des Implantats nach Art der Figuren 1 und 2, wobei hier im Unterschied zu den Figuren 1 und 2 der Implantatkörper mittig über eine einzelne Verbindungsstelle 4 mit dem Hauptgerüst 1 bzw. den beiden Hauptgerüstteilen 1a, 1b verbunden ist.

Weiterhin können die Seitenarme 2 an ihren freien Enden auch miteinander verbunden sein. Fig. 4 zeigt diesbezüglich eine Abwandlung der in den Figuren 1 und 2 gezeigten

Ausführungsform, wobei hier die Enden 2a je zweier benachbarter Seitenarme 2 des proximalen Hauptgerüstteiles 1a, die zur gleichen Seite hin vom proximalen Hauptgerüstteil 1a abgehen, über eine Strebe 5 miteinander verbunden sind. Entsprechend ist vorgesehen, dass die Enden 2a je zweier benachbarter Seitenarme 2 des distalen Hauptgerüstteiles 1b, die zur gleichen Seite hin vom distalen Hauptgerüstteil 1b abgehen, über eine Strebe 5 miteinander verbunden sind. Hierdurch ergibt sich insbesondere auf beiden Seiten der beiden Hauptgerüstteile 1a, 1b eine durchgängige Verbindung der Enden 2a der Seitenarme 2 über Streben 5.

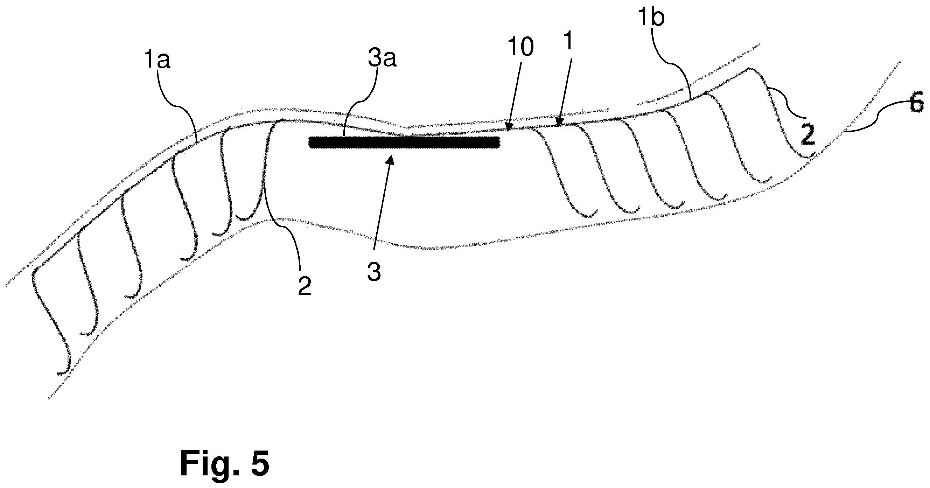

Figur 5 zeigt ein Implantat 3 nach Art der Figuren 1 und 2 implantierten Zustand in einem Gefäß 6 mit gekrümmten Verlauf, wobei erkennbar ist, dass sich die Haltestruktur 10 in vorteilhafter Weise an den Verlauf des Gefäßes 6 anpassen lässt.

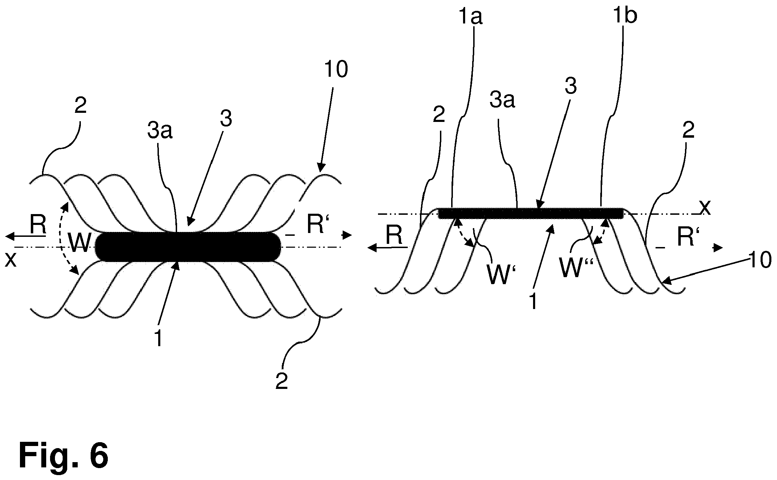

Schließlich besteht gemäß Figur 6 auch die Möglichkeit, auf ein Hauptgerüst 1 zu verzichten bzw. den Implantatkörper 3a selbst als Hauptgerüst 1 für die Haltestruktur 10 zu verwenden. In diesem Sinne wird ein proximaler Hauptgerüstteil 1a durch einen proximalen Abschnitt des Implantatkörpers 3a gebildet, wohingegen ein distaler Hauptgerüstteil durch einen distalen Abschnitt des Implantatkörpers 3a gebildet wird. Die Seitenarme 2 können wiederum in der oben beschriebenen Weise vom proximalen bzw. vom distalen Abschnitt 1a, 1b des Implantatkörpers 3a abgehen.

Die Haltestruktur 10 bzw. das Implantat 3 kann sowohl als selbstexandierende Variante als auch mittels Ballondilatation platziert werden. Bei einer Ballondilatation werden die Seitenarme 2 z. B. mit Hilfe eines teilweise nachgiebigen Ballons aufgedehnt, um so diese entsprechend der Anatomie an die Gefäßwand zu drücken. Der Implantatkörper 3a selbst kann mit einem nicht nachgiebigen Ballon platziert werden. Hierbei kann dieses Prozedere nacheinander mit unterschiedlichen Ballons durchgeführt werden oder aber mit Hilfe eines Ballons, der unterschiedliche Festigkeiten in den entsprechenden Abschnitten aufweist.

Es kann vorgesehen sein, den Durchmesser des Ballons an den verschiedenen Stellen zu variieren, um zu verhindern, dass der Implantatkörper zu stark in die Gefäßwand gedrückt wird. Der Implantatkörper steht weit mehr in das Gefäßvolumen als die Haltestruktur, so dass das Implantat schon wandständig ist, obwohl die Haltestruktur noch nicht vollständig aufgedehnt wurde.

Die erfindungsgemäße Lösung stellt mit Vorteil eine universelle Haltestruktur 10 bereit, wobei insbesondere mit einer Systemgröße eine vergleichsweise große Anzahl von Gefäßdurchmesser abgedeckt werden kann. Die Erfindung erlaubt ferner eine einfache Fixierung auch in stark konischen Gefäßabschnitten. Weiterhin wird eine Implantatmigration aufgrund der gegenläufigen Seitenarmstruktur vermindert.

Die in der Beschreibung, den Ansprüchen und den Figuren offenbarten Merkmale können sowohl einzeln als auch in beliebiger Kombination miteinander für die Verwirklichung von Ausführungsformen relevant sein.

## Patentansprüche

1. Implantat (3), aufweisend einen Implantatkörper (3a) sowie eine Haltestruktur (10) zum Verankern des Implantats in einem Gefäß (6) eines Patienten, wobei die Haltestruktur (10) dazu konfiguriert ist aus einem komprimierten Zustand in einen expandierten Zustand gebracht zu werden, wobei dass die Haltestruktur (10) ein Hauptgerüst (1) sowie mit dem Hauptgerüst (1) verbundene Seitenarme (2) aufweist.

2. Implantat nach Anspruch 1, wobei der jeweilige Seitenarm (2) in dem expandierten Zustand der Haltestruktur (10) einen Kreisbogen bildet, oder dass in dem expandierten Zustand der Haltstruktur (10) eine Projektion des jeweiligen Seitenarmes (2) auf eine Ebene einen Kreisbogen bildet.

3. Implantat nach Anspruch 1 oder 2, wobei die Haltestruktur (10) eine Mehrzahl an Paaren von Seitenarmen (2) aufweist, wobei in dem expandierten Zustand der Haltestruktur (10) eine Projektion des jeweiligen Seitenarmpaares auf eine Ebene einen Kreisbogen mit einem Mittelpunktswinkel (W) von zumindest 180° bildet.

4. Implantat nach einem der vorhergehenden Ansprüche, wobei das Hauptgerüst (1) ein proximales Hauptgerüstteil (1a) und ein distales Hauptgerüstteil (1b) aufweist, wobei eine Anzahl der Seitenarme (2) mit dem proximalen Hauptgerüstteil (1a) verbunden ist und von diesem absteht, und wobei eine restliche Anzahl der Seitenarme (2) mit dem distalen Hauptgerüstteil (1b) verbunden ist und von diesem absteht.

5. Implantat nach Anspruch 4, wobei die beiden Hauptgerüstteile (1a, 1b) jeweils in einer Richtung (R, R') vom Implantatkörper (3a) abstehen oder über den Implantatkörper (3a) hinausragen, wobei die beiden Richtungen (R, R') unterschiedlich sind, insbesondere einander entgegengesetzt.

6. Implantat nach Anspruch 4 oder 5, wobei die mit dem proximalen Hauptgerüstteil (1a) verbundenen Seitenarme (2) unter einem Winkel (W') vom proximalen Hauptgerüstteil (1a) abstehen, der in einem Bereich von 45° bis 90° liegt, und/oder dass die mit dem distalen Hauptgerüstteil (1b) verbundenen Seitenarme (2) unter einem Winkel (W") vom distalen Hauptgerüstteil (1b) abstehen, der in einem Bereich von -90° bis -45° liegt.

7. Implantat nach einem der vorhergehenden Ansprüche, wobei der Implantatkörper (3a) längserstreckt ausgebildet ist und sich entlang einer Längsachse (x) erstreckt.

8. Implantat nach einem der vorhergehenden Ansprüche, wobei der Implantatkörper (3a) ein proximales Ende (30) und ein distales Ende (32) aufweist, wobei insbesondere die beiden Enden (30, 32) über einen mittleren Abschnitt (31) des Implantatkörpers (3a) miteinander verbunden sind

9. Implantat nach Anspruch 8, wobei das proximale Hauptgerüstteil (1a) mit dem proximalen Ende (30) des Implantatkörpers (3a) über eine proximale Verbindungstelle (4a) verbunden ist, und/oder dass der distale Hauptgerüstteil mit dem distalen Ende des Implantatkörper über eine distale Verbindungstelle (4b) verbunden ist.

10. Implantat nach Anspruch 9, wobei die beiden Verbindungstellen (4a, 4b) in einer Ebene mit einer größten Seitenfläche des Implantatkörpers (3a) liegen.

11. Implantat nach Anspruch 8, wobei das distale und der proximale Hauptgerüstteil (1a, 1b) über eine Verbindungstelle (4) mit dem mittleren Abschnitt (31) des Implantatkörpers (3a) verbunden sind.

12. Implantat nach einem der vorhergehenden Ansprüche, wobei das proximale Hauptgerüstteil (1a) durch einen längs erstreckten Arm gebildet ist, und/oder dass das distale Hauptgerüstteil (1b) durch einen längs erstreckten Arm gebildet ist.

13. Implantat nach einem der Ansprüche 1 bis 8, wobei das Hauptgerüst (1) durch den Implantatkörper (3a) gebildet ist.

14. Implantat nach einem der vorhergehenden Ansprüche, wobei der jeweilige Seitenarm (2) ein dem Hauptgerüst (1) gegenüberliegendes Ende (2a) aufweist, wobei zumindest die Enden (2a) zweier benachbarter Seitenarme (2) über eine Strebe (5) miteinander verbunden sind.

15. Implantat nach einem der vorhergehenden Ansprüche, wobei der Implantatkörper (3a) einen Drucksensor (3b) aufweist.
